# EUROPÄISCHE PATENTSCHRIFT

(11) **EP 0 578 997 B1**
(45) Veröffentlichungstag und Bekanntmachung des Hinweises auf die Patenterteilung: **18.12.1996**
(21) Anmeldenummer: 93109894.1
(22) Anmeldetag: 21.06.1993
(51) Int. Cl.: A61B 17/22, A61B 17/00, A61B 19/00

(54) **Laparoskopiebeutel**
Laparoscopy bag
Poche de laparoscopie

(30) Priorität: 25.06.1992 DE 4220785
(43) Veröffentlichungstag der Anmeldung: 19.01.1994
(73) Patentinhaber: Auweiler, Udo, Dr., D-51429 Bergisch-Gladbach (DE)
(72) Erfinder: Auweiler, Udo, Dr., D-51429 Bergisch-Gladbach (DE)
(74) Vertreter: Funck-Hartherz, Anna-Eleonore, Dipl.-Phys.

(56) Entgegenhaltungen:
- EP-A- 0 499 243
- EP-A- 0 537 533

## Beschreibung

Bei endoskopischen Operationen (Laparoskopie) wird der Bauchraum nicht - wie bekannt - über einen Bauchschnitt eröffnet, sondern die notwendigen Instrumente sowie die Optik werden durch einen durch die Bauchdecke gestoßenen Trokar eingeführt, wobei die Instrumente außerhalb des Bauchraumes bedient werden. Dieses Operationsverfahren ist weniger traumatisierend für die Patienten als die bisher durchgeführten Operationen, bei denen der Bauchraum eröffnet wird. Daraus resultiert eine geringere Belastung für den Patienten. Er hat weniger Schmerzen, die Wunde heilt schneller, und dementsprechend ist auch die Krankenhausverweildauer geringer. Dieses Operationsverfahren stößt jedoch dann an Grenzen, wenn größere Tumore oder Gewebeteile aus dem Bauchraum zu entnehmen sind. Sofern diese nicht in ihrer Gänze durch den Trokar entnommen werden können, muß der Tumor und das entzündliche Gewebe im Bauchraum zerkleinert werden, was zu dessen Verunreinigung führt und insbesondere bei malignen Tumoren eine Gefährdung für den Patienten durch Metastasierung darstellt.

Um diese Problematik zu beheben, sind bereits sogenannte Laparoskopiebeutel aus einem flexiblen und haltbaren Material bekanntgeworden, die über Zugschnüre am oberen offenen Ende verschließbar sind. Dieser Beutel wird an den Schnüren durch den Trokar in den Bauchraum gebracht, im Bauchraum geöffnet und dort das zu entfernende Gewebe in den Beutel gebracht. Danach wird der Beutel über die Schnüre verschlossen und mit dem Trokar das obere, zu öffnende Ende aus der Bauchdecke herausgezogen. Danach wird das Gewebe zerkleinert und einzeln aus der Tüte entnommen, worauf der Beutel entfernt wird. Dieser Laparoskopiebeutel bringt zwar schon eine erhebliche Verbesserung insofern, als - theoretisch zumindest - keine Verunreinigung in den Bauchraum gelangen kann, jedoch ist die Entnahme des entfernten und zerstückelten Gewebes kompliziert und zeitraubend. Außerdem besteht die Gefahr, daß bei Entnahme des zerkleinerten Gewebes doch Teile in die Gefäßbahn kommen, was bei malignen Tumoren zu einer Metastasierung führen könnte.

Es sind ferner bereits schlauchartige Laparoskopiebeutel bekannt geworden, die mit entfernten und zerkleinerten Gewebestücken gefüllt und durch einen Trokar beseitigt werden. Diese Beutel sind mit einer Lasche versehen, über die der Beutel herausgezogen wird. Diese Beutel haben den entscheidenden Nachteil, daß kein Arbeitsteil vorgesehen ist, in dem operiert wird und die Gewebeteile gefahrlos zerkleinert werden können. Eine Verunreinigung des Bauchraumes kann somit nicht ausgeschlossen werden.

Die vorliegender Erfindung zugrundeliegende Aufgabe besteht deshalb in einem Laparoskopiebeutel, der eine Entnahme des entfernten und zerkleinerten Gewebes durch den Trokar bei geschlossenem Beutel in toto, also eine Entnahme des gewebegefüllten geschlossenen Beutels zuläßt, so daß die Gefahr einer Eindringung der Gewebeteile in die Gefäße ausgeschlossen ist.

Diese Aufgabe wird durch einen Laparoskopiebeutel gemäß Anspruch 1 gelöst. Der Laparoskopiebeutel besteht aus einem Arbeitsteil, welches groß genug ist, um zu entfernende Teile gut aufnehmen zu können. Das darin zerstückelte Gewebe fällt in den schlauchförmigen Teil hinein oder aber wird nach Schließen und Einziehen in den Trokar in dieses schlauchförmige Reservoir hineingedrückt wird, so daß der Beutel en bloc entfernt werden kann. Das schlauchförmige als Reservoir dienende Teil ist im Durchmesser geringfügig kleiner als der des Trokars. Der schlauchförmige Teil kann als gerader Schlauch oder als Faltenbalg konzipiert sein.

Weitere Einzelheiten der Erfindung und die Handhabung werden anhand der Zeichnungen erläutert. Dabei zeigt
- Fig. 1: eine schematische Darstellung des Laparoskopiebeutels,
- Fig. 2: eine Modifikation des Schlauchbeiles,
- Fig. 3: eine weitere Modifikation des Laparoskopiebeutels,
- Fig. 4: erläutert die Anordnung des Trokars und des Beutels im Bauchraum
- Fig. 5: erläutert die Entnahme des Beutels nach Anordnung und Zerkleinerung des zu entfernenden Gewebes.

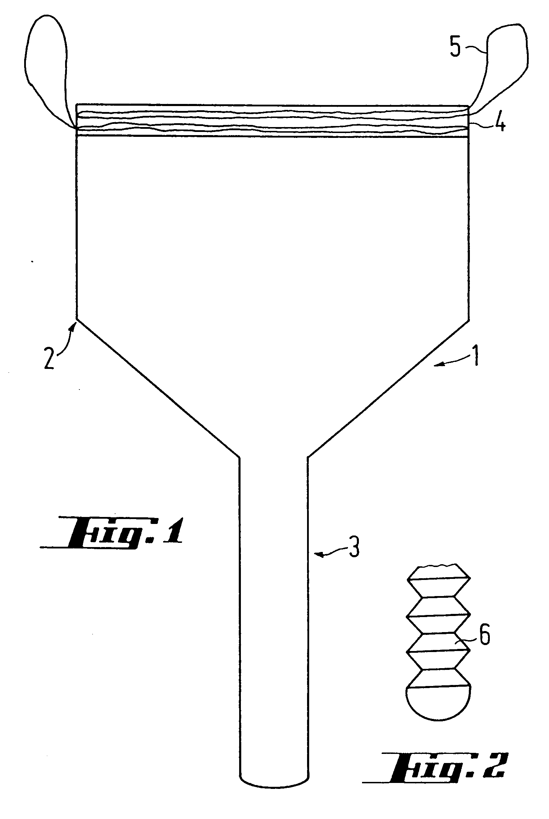

Fig. 1 zeigt die schematische Darstellung eines Laparoskopiebeutels 1 gemäß der Erfindung. Der Beutel 1 teilt sich in den Arbeitsteil 2 sowie das schlauchförmige Reservoir 3. Der Arbeitsteil 2 ist im Querschnitt kreisförmig ausgebildet und läuft trichterförmig in das schlauchartige Reservoir ein. Der obere Rand 4 des Beutels 1 ist mit Zugschnüren 5 zum Verschließen ausgestattet. Der Durchmesser des Reservoirs 3 ist geringfügig kleiner als der des bei der Operation verwendeten Trokars ausgebildet. In Fig. 1 ist ein gerader Schlauch als Reservoir verwendet. Der Laparoskopiebeutel 1 besteht aus einer Klarsichtfolie mit einer Eigenfestigkeit, so daß dieser im Bauchraum geöffnet werden kann und in der geöffneten Stellung verbleibt. Das Material soll antistatisch, pyrogenfrei, giftfrei und sterilisierbar sein. Der Arbeitsraum des Arbeitsteiles 2 hat üblicherweise einen Durchmesser von 15 cm, jedoch sind Beutel auch je nach dem zu entfernenden Gewebeteil in anderen Abmessungen denkbar.

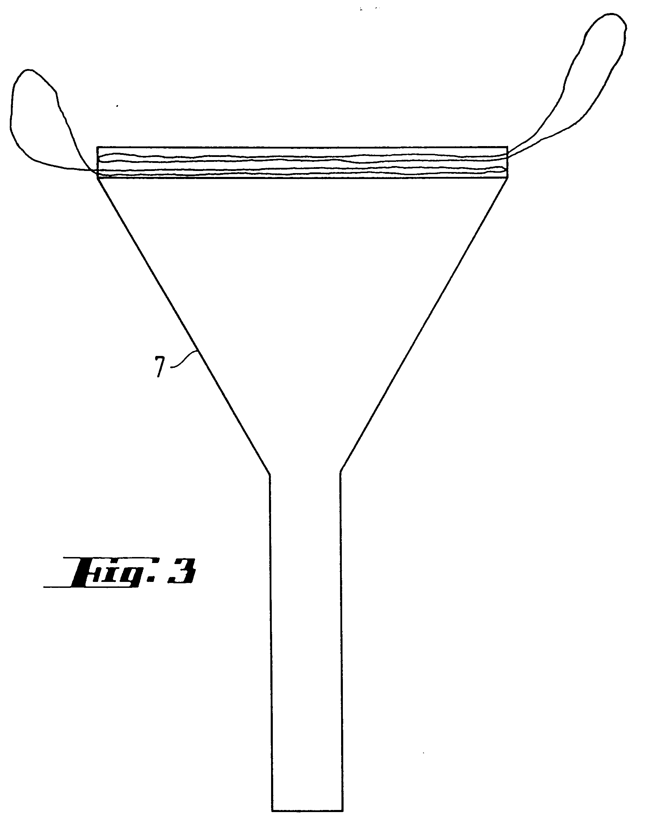

Fig. 2 zeigt eine Modifikation des Reservoirs, das dabei als Faltenbalg 6 ausgebildet ist, um das zur Verfügung stehende Reservoirvolumen im Bedarfsfall zu vergrößern.

Fig. 3 zeigt einen Laparoskopiebeutel, dessen Arbeitsteil im Ganzen trichterförmig ausgebildet ist und ansonsten mit der Ausführung gemäß Fig. 1 übereinstimmt. Diese Modifikation eignet sich für kleinere Gewebeteile und leicht zu zerlegende von weicher Konsistenz.

Der Laparoskopiebeutel gemäß der Erfindung ist vor Gebrauch entweder von der Reservoirseite her zu einer Rolle aufgerollt und kann so einfach durch den Trokar in den Bauchraum eingeführt werden. Eine andere Möglichkeit besteht darin, den Arbeitsteil in das schlauchförmige Reservoir 3 zu versenken, wobei die Zugschnüre frei oben aus dem Reservoir herausragen. Im Bauchraum läßt sich der Beutel mit zwei Instrumenten dann leicht entfalten.

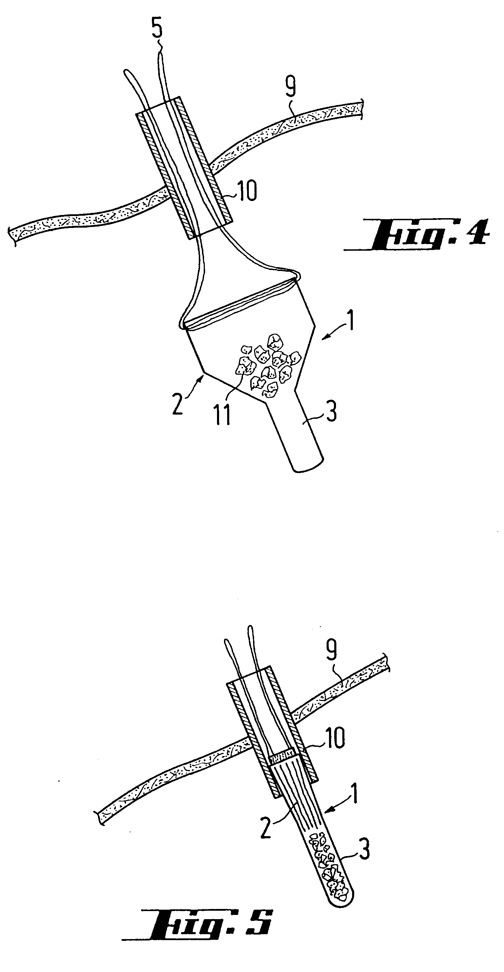

Anhand der Figuren 4 und 5 wird die Anwendung des Laparoskopiebeutels erläutert. Nach erfolgter diagnostischer Laparoskopie wird ein Trokar, vorzugsweise von 2 cm, durch die Bauchdecke 9 appliziert. Über diesen Trokar wird die sterile gefaltete Tüte in den Bauchraum eingeführt und mit zwei stumpfen Instrumenten geöffnet. Diese Phase ist in Fig. 4 dargestellt, wobei die beiden Zugschnüre aus dem Trokar herausragend dargestellt sind. Es besteht natürlich auch die Möglichkeit, daß die Zugschnüre mitsamt des Beutels in den Bauchraum gelangen und die Zugschnüre über entsprechende Instrumente betätigt werden. Danach wird der zu entfernende Tumor oder das zu entfernende Gewebe abgetrennt und in den Arbeitsteil des Beutels plaziert. Der Tumor läßt sich dann in dem Beutel mit Schere oder Skalpell zerkleinern, wobei darauf geachtet werden muß, daß keine Beschädigung des Beutels erfolgt und kein abgetrenntes Gewebeteil in den Bauchraum gelangt. Ist das Gewebe 11 so weit zerkleinert, daß es von dem Reservoir 3 aufgenommen werden kann, wird der Beutel über die beiden Zugschnüre geschlossen. Danach wird der Beutel über die Zugschnüre durch den Trokar gezogen. Sobald die Tüte den Trokar erreicht, wird sie zusammengepreßt, und durch die erfindungsgemäße Form des Beutels gelangt das zerkleinerte Gewebe - sofern es nicht bereits in das Reservoir gerutscht ist - aus dem Arbeitsraum des Beutels unter Druck in das Reservoir. Diese Stellung ist in Fig. 5 gezeigt. Danach kann die gesamte Tüte en bloc mit dem Gewebe im Reservoir problemlos durch den Trokar herausgezogen werden. Auf diese Weise ist gesichert, daß keinerlei Gewebeteile in den Bauchraum kommen können und jegliche Kontamination des Bauchraumes verhindert wird.

## Patentansprüche

1. Laparoskopiebeutel aus einem flexiblen Material mit an der Öffnung angebrachten Zugschnüren, insbesondere für die Verwendung in einem Trokar,
**dadurch gekennzeichnet**,
daß der Beutel (1) aus einem Arbeitsteil (2) besteht, an den sich ein schlauchförmiger Teil (3) anschließt.

2. Beutel nach Anspruch 1,
**dadurch gekennzeichnet**,
daß der Arbeitsteil (2) des Beutels (1) in ein schlauchförmiges Teil (3, 6) ausläuft.

3. Beutel nach Anspruch 1 oder 2,
**dadurch gekennzeichnet**
daß der Beutel (1) aus einem im Querschnitt runden Arbeitsteil (2) besteht, das sich am unteren Ende konisch in ein Schlauchteil (3, 6) verjüngt.

4. Beutel nach Anspruch 1 oder 2,
**dadurch gekennzeichnet**
daß der Arbeitsteil (2) des Beutels (1) trichterförmig ist und in ein Schlauchteil (3, 6) ausläuft.

5. Beutel nach einem oder mehreren der vorhergehenden Ansprüche,
**dadurch gekennzeichnet**,
daß der Schlauchteil (3, 6) des Beutels (1) als Faltenbalg ausgebildet ist.

6. Beutel nach einem oder mehreren der vorhergehenden Ansprüche,
**dadurch gekennzeichnet,**
daß der Durchmesser des Schlauchteils (3, 6) gleich oder kleiner ist als der Durchmesser des Trokars.

## Claims

1. Laparoscopy bag made of a flexible material with traction laces fastened to the opening, in particular for use in a trocar,
characterized in that
bag (1) consists of a working part (2) ending in a hose-shaped part (3).

2. Bag according to Claim 1,
characterized in that
the working part (2) of bag (1) ends in a hose-shaped part (3,6).

3. Bag according to Claims 1 or 2,
characterized in that
bag (1) consists of a working part (2) having a circular cross section, its bottom end tapering off to form a conical hose (3,6).

4. Bag according to Claims 1 or 2,
characterized in that
the working part (2) of bag (1) is funnel-shaped and ends in a hose (3,6).

5. Bag according to one or several of the foregoing Claims,
characterized in that
the hose (3,6) of bag (1) is formed by a folding bellows.

6. Bag according to one or several of the foregoing Claims,
characterized in that
the diameter of the hose (3,6) is equal or smaller than that of the trocar.

## Revendications

1. Poche de laparoscope composée d'un matériau flexible avec tirettes attachées à l'orifice, en particulier pour l'utilisation dans un trocart,
caractérisée en ce que
la poche (1) est constituée d'une pièce de travail (2) à laquelle est raccordée une pièce en forme de tuyau.

2. Poche selon la revendication 1,
caractérisée en ce que
la pièce de travail (2) de la poche (1) aboutit dans un tuyau (3).

3. Poche selon les revendications 1 ou 2,
caractérisée en ce que
la poche (1) est constituée d'une pièce de travail (2) à section transversale circulaire, qui, à sa partie inférieure se réduit en forme de cone et aboutit dans une pièce en forme de tuyau

4. Poche selon les revendications 1 ou 2,
caractérisée en ce que
la pièce de travail (2) de la poche (1) a la forme d'un entonnoir et aboutit dans un tuyau (3,6).

5. Poche selon une ou plusieurs des revendications précédentes,
caractérisée en ce que
la partie tuyau (3,6) de la poche (1) a la forme d'un soufflet à plis.

6. Poche selon une ou plusieurs des revendications précédentes,
caracterisées en ce que
le diamètre de la partie tuyau (3,6) est égal ou inférieur au diamètre du trocart.
